Europäisches Patentamt

(19) European Patent Office  (11) Veröffentlichungsnummer: **0 005 148**
**B1**
Office européen des brevets

(12) EUROPÄISCHE PATENTSCHRIFT

(45) Veröffentlichungstag der Patentschrift:  (51) Int. Cl.³: **C 08 B 31/00**, C 08 B 33/00,
25.11.81                                                **G 01 N 33/52**

(21) Anmeldenummer: 79100499.7

(22) Anmeldetag: 20.02.79

(54) Alpha-Amylasesubstrat und Verfahren zu seiner Herstellung.

(30) Priorität: 02.05.78 DE 2819298

(43) Veröffentlichungstag der Anmeldung:
14.11.79 Patentblatt 79/23

(45) Bekanntmachung des Hinweises auf die Patenterteilung:
25.11.81 Patentblatt 81/47

(84) Benannte Vertragsstaaten:
BE CH DE FR GB IT LU NL SE

(56) Entgegenhaltungen:
DE-A-2 408 008
GB-A-1 381 380
US-A-2 977 356
US-A-4 066 509
CHEMICAL ABSTRACTS, volume 85,
1976, November 22, No. 21, Ref.
155496v, Seite 174
COLUMBUS (OHIO)
CHEMICAL ABSTRACTS, volume 89,
1978, December 4, no. 23, Ref.
192896d, Seite 230

(73) Patentinhaber: BOEHRINGER MANNHEIM GMBH,
Sandhofer Strasse 112-132 Postfach 31 01 20,
D-6800 Mannheim 31-Waldhof (DE)

(72) Erfinder: Batz, Hans-Georg, Dr., Kreuzeckstrasse 13,
D-8132 Tutzing (DE)
Erfinder: Rauscher, Eill, Dr., Guardinistrasse 71,
D-8000 München 70 (DE)
Erfinder: Weimann, Günter, Dr., Waxensteinstrasse 20,
D-8132 Tutzing (DE)
Erfinder: Wahlefeld, August Wilhelm, Dr., Am Betberg 34,
D-8120 Weilheim (DE)
Erfinder: Gruber, Wolfgang, Dr., Am Oberanger 7,
D-8132 Tutzing-Unterzeismering (DE)

(74) Vertreter: Weickmann, Heinrich, Dipl.-Ing. et al,
Postfach 860 820 Möhlstrasse 22,
D-8000 München 86 (DE)

## Alpha-Amylasesubstrat und Verfahren zu seiner Herstellung

Die Erfindung betrifft Substrate auf Basis von teilchenförmiger, mit bestimmbaren Gruppen derivatisierter Stärke bzw. Amylose für die Bestimmung der $\alpha$-Amylase und Verfahren zu deren Herstellung.

Die Bestimmung des $\alpha$-Amylasespiegels im Serum ist ein wichtiger klinischer Parameter für die Bauchspeicheldrüsenfunktion.

$\alpha$-Amylase spaltet bekanntlich die 1,4-glykosidische Bindung von Stärke und Stärkederivaten. Dabei muß der Angriff nicht vom Kettenende erfolgen, sondern kann auch in der Kettenmitte eines vernetzten Stärkemoleküls beginnen. Unlösliche Stärke wird hierdurch zu löslichen Stärkebruchstücken, unverzeigte Ketten werden letztlich bis zur Maltose gespalten. Die Geschwindigkeit, mit der dieser Abbau erfolgt, ist ein Maß für die Menge vorhandener angreifender Amylase. Verwendet man gefärbte unlösliche Stärke, so entstehen angefärbte lösliche Bruchstücke und man kann so den Grad der Spaltung von der entstandenen Färbung der Lösung ableiten (Experientia 23 [1967] 805). Als Farbstoffe werden hier Reaktionsfarbstoffe vorgeschlagen, wie sie auch zur Anfärbung von Cellulosefasern verwendet werden.

Da nun Stärke nicht absolut unlöslich und daher nur schlecht zu verarbeiten (wie etwa anzufärben) ist, wurde in der DE-OS 1 901 517 vorgeschlagen, durch Vernetzung von löslicher Stärke (z. B. mit Epichlorhydrin) ein absolut unlösliches Gel herzustellen, dieses dann zu zerkleinern und anzufärben.

Die Genauigkeit dieses Amylase-Bestimmungssystems beruht letztlich darauf, daß dem Enzym ein so großer Überschuß des Substrats angeboten wird, daß auch nach Ende (Stoppen) der Reaktion überschüssiges Substrat vorliegt, d. h., daß das Enzym während der Reaktion stets optimal spalten kann. Das bedeutet aber weiterhin, daß für vergleichende Messungen nicht nur gleiche Substratmengen, sondern auch vergleichbar viel Oberfläche dieses Substrats angeboten werden muß, ebenso wie ein vergleichbares und gleichmäßiges Netzwerk. Bei dem Substrat nach DE-OS 1 901 517 werden der Amylase zwar vergleichbare Oberflächen pro Gewichtseinheit (durch Brechen des Geles in etwa gleich große Scherben mittels eines Siebes) angeboten, ein stets gleicher Vernetzungsgrad ist dagegen nicht reproduzierbar zu erreichen, da zum einen bei der Spaltung der an sich schon nicht einheitlichen Stärke eine Mischung von löslichen, im Mittel von Ansatz zu Ansatz unterschiedlich langen Stärkebruchstücken entsteht, zum anderen bei der anschließenden Vernetzung, bedingt durch den sogenannten Trommsdorf-Effekt, ein ungleichmäßiges vernetztes Produkt zu erwarten ist.

Der Trommsdorf-Effekt beschreibt die sich selbst beschleunigenden, durch Entropieverlust exotherm ablaufende Vernetzungsreaktion, die nur sehr schwer unter Kontrolle zu halten ist.

Aus diesen Gründen ist das, wie oben beschrieben, hergestellte Substrat von Charge zu Charge verschieden. Auch treten Probleme mit der Linearität der Absorption bei steigender Enzymkonzentration auf (Clin. Chim. Acta [1973] 233). Daher muß pro Packung eine Eichkurve erstellt werden, was zusätzlichen Aufwand bedeutet.

Der Erfindung liegt daher die Aufgabe zugrunde, die Nachteile dieser bekannten vernetzten Substrate der $\alpha$-Amylase auf Basis von Stärke zu beseitigen und ein Substrat zu schaffen, welches wesentlich gleichmäßiger ist, und zwar sowohl innerhalb einer Charge als auch von Charge zu Charge, eine bessere Linearität der Absorption ergibt und das Erstellen neuer Eichkurven unnötig macht.

Gelöst wird diese Aufgabe erfindungsgemäß durch ein Substrat auf Basis von teilchenförmiger, mit bestimmbaren Gruppen derivatisierter Stärke für die Bestimmung der $\alpha$-Amylase, welches dadurch gekennzeichnet ist, daß es

a) aus oberflächlich vernetzten Stärkekörnchen mit geregeltem verringertem Quellvermögen besteht, die mit 0,0009 bis 0,0027 Mol eines difunktionellen Vernetzungsmittels je 100 g nichtgequollene Stärkekörnchen vernetzt sind und einen Durchmesser zwischen 0,01 und 0,1 mm aufweisen, oder

b) aus oberflächlich vernetzten Amylosekörnchen mit geregeltem verringertem Quellvermögen besteht, die mit 0,02 bis 0,07 Mol eines difunktionellen Vernetzungsmittels je 100 g nichtgequollene Amylosekörnchen vernetzt sind und einen Durchmesser zwischen 0,05 und 0,20 mm aufweisen.

Die Vernetzung von ungelatinisierter Stärke mit Hilfe von Ätherbildungsmitteln ist bereits aus der US-PS 2 500 950 bekannt. Die dort beschriebenen Produkte sollen als Nahrungsmittel sowie im Rahmen der Textilausrüstung verwendbar sein. Aus der DE-AS 1 668 515 ist es weiter schon bekannt, derartige durch Vernetzung im Quellvermögen gehemmte Stärkekörnchen mit $\beta$-Amylase, $\alpha$-1,4-Glucosidase oder Phosphorylase abzubauen, um ein Stärkeabbauprodukt zu erhalten, welches als Bestandteil von dosenkonservierten Nahrungsmitteln diesen verbesserte Tieftemperaturstabilität verleiht. Irgendwelche Hinweise, daß oberflächlich vernetzte derivatisierte Stärke- oder Amylosekörnchen des angegebenen Durchmessers durch $\alpha$-Amylase besonders gleichmäßig angegriffen werden und sich daher für analytische Zwecke eignen, lassen sich daraus nicht entnehmen. Überraschenderweise liefert nämlich die an sich inhomogene Stärke nach dem Vernetzen durch das Aufquellen ein Substrat, welches wesentlich gleichmäßiger abgebaut wird und daher eine überlegene Eignung als Substrat für die α-Amylase besitzt. Es wird angenommen, daß die an sich schon vernetzte Amylopektinhülle im äußeren Teil des Stärkekorns bei dem unten beschriebenen Verfahren verstärkt wird, so daß der α-Amylase ein an der normalen Quellung gehindertes und somit unter einem gewissen Innendruck stehendes Substrat angeboten wird.

Das erfindungsgemäße Verfahren zur Herstellung des oben definierten Substrats auf Basis von Stärkekörnchen ist dadurch gekennzeichnet, daß von nicht gequollenen Stärkekörnchen mit einem Durchmesser zwischen 0,01 und 0,20 mm ausgegangen wird, die in einer Lösung mit 0,0009 bis 0,0027 Mol eines difunktionellen Vernetzungsmittel je 100 g nicht gequollene Stärke suspendiert werden, bis das Vernetzungsmittel verbraucht ist und danach in an sich bekannter Weise die Körnchen mit bestimmbaren Gruppen derivatisiert werden.

In analoger Weise ist das erfindungsgemäße Verfahren zur Herstellung des oben definierten Substrats auf Basis von Amylosekörnchen dadurch gekennzeichnet, daß von nicht gequollenen Amylosekörnchen mit einem Durchmesser zwischen 0,1 und 0,20 mm ausgegangen wird, die in einer Lösung mit 0,02 bis 0,07 Mol eines difunktionellen Vernetzungsmittel je 100 g nicht gequollene Amylosekörnchen suspendiert werden, bis das Vernetzungsmittel verbraucht ist und danach in an sich bekannter Weise die Körnchen mit bestimmbaren Gruppen derivatisiert werden.

Geeignete difunktionelle Vernetzungsmittel sind beispielsweise aliphatische Halogenide, wie Propylendichlorid, Dichlorpentan, Äthylendibromid, Glycerindichlorhydrin, Dichlorbutan, ätherbildende Epoxyhalogenverbindungen, wie Epichlorhydrin und Epibromhydrin, Cyanurchlorid, Phosphoroxychlorid, Methaphosphate und Polymethaphosphat, Aldehyde, wie Formaldehyd, Glutardialdehyd, Acrolein, Bernsteinsäureanhydrid und ähnliche.

Wie oben bereits erwähnt, wird bei Anwendung des Verfahrens auf Stärkekörnchen eine Vernetzungsmittelmenge von 0,0009 bis 0,0027 Mol je 100 g nichtgequollene Stärkekörnchen verwendet, wobei der Durchmesser dieser Körnchen am vorteilhaftesten zwischen 0,01 und 0,1 mm liegt. Bei Amylosekörnchen dagegen werden die besten Ergebnisse mit 0,02 bis 0,07 Mol Vernetzungsmittel je 100 g nichtgequollene Amylosekörnchen erzielt, wobei letztere am besten einen Durchmesser zwischen 0,05 und 0,20 mm aufweisen. Diese Unterschiede rühren daher, daß bei dem Stärkekorn die natürliche Amylopektinhülle an sich bereits vernetzt ist und daher für die Erzielung der gewünschten gebremsten Quellbarkeit relativ wenig Vernetzungsmittel erforderlich ist. Bei zu geringer Vernetzungsmittelmenge ist die Quellbarkeit zu groß, bei zu hoher Vernetzungsmittelmenge werden die Körnchen zu stabil und es sind nicht mehr genügend freie Amyloseketten vorhanden, die durch die α-Amylase angegriffen werden

können. Die Amylose hingegen ist von vornherein nicht vernetzt, so daß man zur Herstellung des erfindungsgemäßen Substrats eine mehr als 10fach so große Vernetzermenge verwendet. Während bei der Stärke erfindungsgemäß die Quellung gebremst wird, erhält man bei den kristallinen, im kalten Wasser unlöslichen Amylosepartikelchen durch die Vernetzung überhaupt erst quellbare Produkte. Da bei technischer Amylose häufig noch gewisse Mengen an Amylopektin enthalten sind, ist es zweckmäßig, den Amylopektingehalt zu bestimmen und daraus die benötigte Vernetzermenge abzuleiten.

Als bestimmbare Gruppen, mit denen das Substrat derivatisiert ist, kommen in erster Linie Farbstoffe oder farbstoffbildende Gruppen, also solche, die erst nachträglich mit einer weiteren Komponente zum Farbstoff umgesetzt werden, in Betracht. Die Farbstoffe können im sichtbaren Licht oder bei nichtsichtbaren Wellenlängen absorbieren. Auch Fluoreszenzfarbstoffe sind geeignet. Ferner können radioaktiv oder enzymatisch bestimmbare Gruppen eingeführt werden. Als besonders geeignet erwies sich Cibacronblau 3 GA. Als ungeeignet erwiesen sich Farbstoffe mit einem Diazosystem aufgrund ihrer Wechselwirkung mit Polysacchariden sowie ihrer Hydrophobie.

Außer dem oben beschriebenen bevorzugten Farbstoff erwiesen sich andere chemisch ähnlich aufgebaute Farbstoffe als ebenso gut verwendbar, sie enthielten jedoch in der Praxis vielfach Naphthalinsulfonsäure als Zusatz und letztere inhibiert die α-Amylase schon in sehr geringen Mengen.

Auch für andere bestimmbare Gruppen, die sich zur Derivatisierung des Substrats von der chemischen Struktur her eignen, ist es wichtig, daß sie die α-Amylase selbst nicht inhibieren, was in jedem Fall leicht durch einen einfachen Vorversuch festgestellt werden kann. Die Derivatisierung selbst erfolgt nach dem Fachmann bekannten Methoden, beispielsweise wie sie in der Baumwollfärberei üblich sind, und brauchen hier nicht näher beschrieben zu werden.

Das erfindungsgemäße Substrat für die α-Amylasebestimmung wird zweckmäßig in Mengen zwischen etwa 5 mg/ml und 150 mg/ml eingesetzt. Bevorzugt werden Mengen zwischen etwa 20 und etwa 50 mg/ml, da hierbei unter allen Bedingungen, also auch bei pathologischen Seren ein ausreichend großer Substratüberschuß zur Verfügung steht.

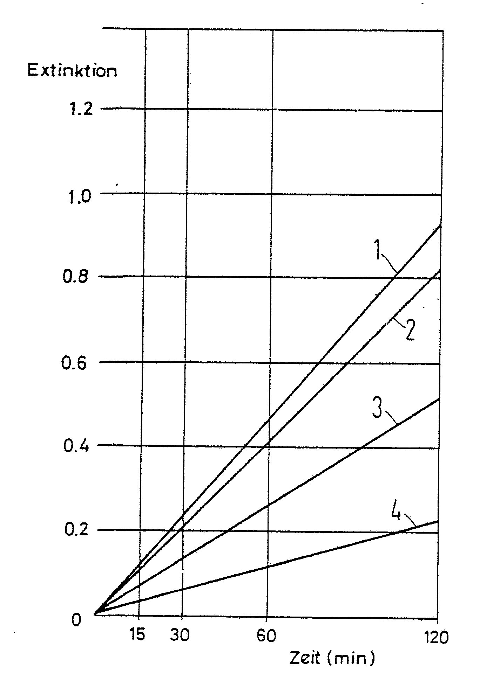

In der beigefügten Zeichnung wird das erfindungsgemäße Reagens hinsichtlich der pro Zeiteinheit entwickelten Extinktion bei gleicher Subtratmenge und auch sonst identischen Bedingungen mit zwei handelsüblichen Amylasesubstraten auf Basis angefärbter Stärke verglichen. Kurve 1 identifiziert das erfindungsgemäße Substrat auf Basis von Stärke, Kurve 2 das erfindungsgemäße Substrat auf Basis von Amylose, Kurve 3 ein handelsübliches Substrat, welches durch Vernetzung löslicher Stärke und anschließende Anfärbung hergestellt wurde, Fig. 4 identifiziert ein handelsübliches Substrat anderen Ursprungs, welches im Prinzip wie das Substrat von Kurve 3 hergestellt wurde. Sämtliche Substrate waren mit Cibachronblau 3 GA angefärbt.

Die in der Zeichnung dargestellten Kurven wurden erhalten, indem jeweils 5,0 ml einer 10%igen Substratsuspension mit 100 µl α-Amylasenorm bei 30°C inkubiert wurden. Zu bestimmten Zeiten wurden 1,0 ml 0,5n NaOH zugesetzt, filtriert und die Extinktion E 578 bei n = 2 gegen Luft gemessen. Wie die Figur zeigt, wird bei den erfindungsgemäßen Substraten bei etwa gleichem Leerwert, wie ihn die Vergleichssubstrate aufweisen, eine fast doppelt so hohe Extinktion erzielt.

Die folgenden Beispiele erläutern die Erfindung weiter.

Beispiel 1

Herstellung von
α-Amylasesubstrat aus Kartoffelstärke

16 g NaOH werden unter Rühren in 6 l Wasser eingetragen, dann werden 1000 g Stärke (mittlere Korngröße 0,01 bis 0,1 mm, gesiebt) eingerührt und langsam 2 ml Epichlorhydrin zugetropft. Der Ansatz wird 15 Stunden bei Raumtemperatur gerührt, danach mit 2n HCl auf pH 7 bis 8 gebracht (etwa 200 ml 2n HCl) und etwa 1 Stunde stehengelassen. Die vernetzte Stärke setzt sich dabei ab, der leicht trübe Überstand wird abdekantiert und verworfen. Das Restvolumen von 2 bis 3 l wird in die etwa 5fache Menge an Methanol unter kräftigem Rühren eingetragen. Die Suspension wird noch 1 Stunde nachgerührt, dann wird der Niederschlag abgesaugt und im Vakuum bei Raumtemperatur getrocknet.

Ausbeute: 0,8 bis 1 kg.

Das Anfärben wird wie folgt durchgeführt: 6 kg Natriumsulfat (wasserfrei) werden unter Rühren und Erwärmen in 30 l Wasser gelöst, dann wird auf 98°C erhitzt und 1 kg der vernetzten Stärke portionsweise eingetragen. Dann werden 0,4 kg Farbstoff Cibacronblau 3 GA, zugegeben, worauf die Lösung noch 10 Minuten bei dieser Temperatur gehalten wird. Hierauf werden 0,92 kg Natriumphosphat · 12 $H_2O$ zugegeben, worauf die Lösung nochmals 15 Minuten bei 95 bis 98°C gerührt wird. Danach läßt man abkühlen und absetzen. Der Ansatz wird abfiltriert oder abzentrifugiert und so lange gewaschen, bis das Filtrat farblos ist. Das gequollene wasserhaltige Produkt wird sodann in etwa die 5fache Volumenmenge an Methanol unter kräftigem Rühren eingetragen. Die entstandene Suspension wird noch 1 Stunde nachgerührt und anschließend abgesaugt. Dann wird mit Aceton nachgewaschen und im Vakuum bei Raumtemperatur getrocknet.

Ausbeute: 1,0 bis 1,1 kg gefärbte vernetzte Stärke.

Beispiel 2

Herstellung des
α-Amylasesubstrats auf Basis von Amylose

Man verfährt wie in Beispiel 1 beschrieben, jedoch werden anstelle der Stärke 1000 g Amylose mit einem mittleren Körnchendurchmesser zwischen 0,05 und 0,20 mm (gesiebt und 25 ml Epichlorhydrin eingesetzt.

Beispiel 3

Durchführung der α-Amylasebestimmung

| Reagentien | Konzentrationen |
|---|---|
| Suspension des Substrats | 20 mg/ml in 20 mMol Natriumphosphatpuffer, enthaltend 50 mMol NaCl, pH 7,0 |
| Natronlauge | 0,5 N |
| Meßbedingungen: | |
| Inkubationstemperatur | 30°C |
| Inkubationszeit | 15 Minuten |
| Meßwellenlänge | 578 nm (550 bis 650 nm) |
| Schichtdecke | 1 cm |

Reagentienleerwert: Anstelle von Probe wird destilliertes Wasser eingesetzt.
In Reaktionsgefäß pipettieren:

| | |
|---|---|
| Suspension (30°C) | 5,0 ml |
| Probe | 0,1 ml |

mischen, genau 15 Minuten bei 30°C inkubieren Reaktion abstoppen durch Zugabe von:

| | |
|---|---|
| NaOH 0,5 N | 1,0 ml |

durch Faltenfilter filtrieren (evtl. zentrifugieren und vorsichtig abgießen)
Extinktion des Überstands messen gegen Reagentienleerwert (RLW).

$$E_{Probe} - E_{RLW} = \Delta E$$

Auswertung über Farb- oder Enzymstandardlösung.

Patentansprüche

1. Substrat auf Basis von teilchenförmiger, mit bestimmbaren Gruppen derivatisierter Stärke für die Bestimmung der α-Amylase, dadurch gekennzeichnet, daß es aus oberflächlich vernetzten Stärkekörnchen mit geregeltem verringertem Quellvermögen besteht, die mit 0,0009 bis 0,0027 Mol eines difunktionellen Vernetzungs-

mittels je 100 g nichtgequellene Stärkekörnchen vernetzt sind und einen Durchmesser zwischen 0,01 und 0,1 mm aufweisen.

2. Substrat auf Basis von teilchenförmiger, mit bestimmbaren Gruppen derivatisierenter Amylose für die Bestimmung der α-Amylase, dadurch gekennzeichnet, daß es aus oberflächlich vernetzten Amylosekörnchen mit geregeltem verringertem Quellvermögen besteht, die mit 0,02 bis 0,07 Mol eines difunktionellen Vernetzungsmittels je 100 g nichtgequollene Amylosekörnchen vernetzt sind und einen Durchmesser zwischen 0,05 und 0,20 mm aufweisen.

3. Substrat nach einem der vorhergehenden Ansprüche, dadurch gekennzeichnet, daß es mit einem Reaktivfarbstoff, der kein Diazo-farbsystem enthält, als bestimmbare Gruppe derivatisiert ist.

4. Verfahren zur Herstellung von einem Substrat nach Anspruch 1, dadurch gekennzeichnet, daß von nicht gequollenen Stärkekörnchen mit einem Durchmesser zwischen 0,01 und 0,10 mm ausgegangen wird, die in einer Lösung mit 0,0009 bis 0,0027 Mol eines difunktionellen Vernetzungsmittel je 100 g nicht gequollene Stärke suspendiert werden, bis das Vernetzungsmittel verbraucht ist und danach in an sich bekannter Weise die Körnchen mit bestimmbaren Gruppen derivatisiert werden.

5. Verfahren zur Herstellung von einem Substrat nach Anspruch 2, dadurch gekennzeichnet, daß von nicht gequollenen Amylosekörnchen mit einem Durchmesser zwischen 0,05 und 0,20 mm ausgegangen wird, die in einer Lösung mit 0,02 bis 0,07 Mol eines difunktionellen Vernetzungsmittel je 100 g nicht gequollene Amylosekörnchen suspendiert werden, bis das Vernetzungsmittel verbraucht ist und danach in an sich bekannter Weise die Körnchen mit bestimmbaren Gruppen derivatisiert werden.

6. Verfahren nach Anspruch 4 oder 5, dadurch gekennzeichnet, daß Epichlorhydrin als Vernetzungsmittel verwendet wird.

7. Verfahren nach Anspruch 4 oder 5, dadurch gekennzeichnet, daß die Körnchen mit Cibachronblau 3 GA angefärbt werden.

## Claims

1. Substrate based on particulate starch derivatised with determinable groups for the determination of α-amylase, characterised in that it consists of superficially cross-linked starch granules with regulated, reduced swellability, which are cross-linked with 0.0009 to 0.0027 mol of a difunctional cross-linking agent per 100 g. of non-swollen starch granules and have a diameter of between 0.01 and 0.1 mm.

2. Substrate based on particulate amylose derivatised with determinable groups for the determination of α-amylase, characterised in that it consists of superficially cross-linked amylose granules with regulated, reduced swellability, which are cross-linked with 0.02 to 0.07 mol of a difunctional cross-linking agent per 100 g. of non-swollen amylose particles and have a diameter of between 0.05 and 0.20 mm.

3. Substrate according to one of the preceding claims, characterised in that it is derivatised with a reactive dyestuff, which does not contain a diazo colour system as determinable group.

4. Process for the preparation of a substrate according to chlaim 1, characterised in that one starts from non-swollen starch granules with an diameter of between 0.01 and 0.10 mm. which are suspended in a solution with 0.0009 to 0.0027 mol of a difunctional cross-linking agent per 100 g. of non-swollen starch until the corss-linking agent is used up an thereafter the granules are derivatised in per se known manner with determinable groups.

5. Process for the preparation of a substrate according to claim 2, characterised in that one starts form non-swollen amylose granules with a diameter of between 0.05 and 0.20 mm. which are suspended in a solution with 0.02 to 0.07 mol of a difunctional cross-linking agent per 100 g. of non-swollen amylose granules until the cross-linking agent is used up and thereafter the granules are derivatised in per se known manner with determinable groups.

6. Process according to claim 4 or 5, characterised in that epichlorohydrine is used as a cross-linking agent.

7. Process according to claim 4 or 5, characterised in that the granules are dyed with Cibacron Blue 3 GA.

## Revendications

1. Substrat à base d'amidon particulaire, transformé en dérivé au moyen de groupes dosables, pour le dosage de l'α-amylase, caractérisé en ce qu'il est constitué de gramules d'amidon réticulés en surface avec un pouvoir gonflant réglé abaissé, qui sont réticulés avec 0,0009 à 0,0027 mole d'un agent réticulant bifonctionnel pour 100 g de granules d'amidon non gonflés et présentent un diamètre compris entre 0,01 et 0,1 mm.

2. Substrat à base d'amylose particulaire, transformé en dérivé au moyen de groupes dosables pour le dosage de l'α-amylase, caractérisé en ce qu'il est constitué de granules d'amylose réticulés en surface avec un pouvoir gonflant réglé abaissé, qui sont réticulés avec 0,02 à 0,07 mole d'un agent réticulant bifonctionnel pour 100 g de granules d'amylose non gonflés et présentent un diamètre compris entre 0,05 et 0,20 mm.

3. Substrat selon l'une des revendications précédentes, caractérisé en ce qu'il est transformé en dérivé au moyen d'un colorant réactif qui ne contient pas de système colorant diazo, en tant que groupe dosable.

4. Procédé pour la préparation d'un substrat selon la revendication 1, caractérisé en ce qu'on part de granules d'amidon non gonflés avec un dimaètre compris entre 0,01 et 0,10 mm qui sont mis en suspension dans une solution avec 0,0009 à 0,0027 mole d'un agent réticulant bifonctionnel pour 100 g d'amidon non gonflé, jusqu'à ce que l'agent réticulant soit consommé et ensuite on transforme, de manière en soi connue, les granules en dérivé au moyen de groupes dosables.

5. Procédé pour la préparation d'un substrat selon la revendication 2, caractérisé en ce qu'on part de granules d'amylose non gonflés avec un diamètre compris entre 0,05 et 0,20 mm qui sont mis en suspension dans une solution avec 0,02 à 0,07 mole d'un agent réticulant bifonctionnel pour 100 g de granules d'amylose non gonflés jusqu'à ce que l'agent réticulant soit consommé et ensuite on transforme, de manière en soi connue, les granules en dérivé au moyen de groupes dosables.

6. Procédé selon la revendication 4 ou 5, caractérisé en ce qu'on utilise de l'épichlorhydrine en tant qu'agent réticulant.

7. Procédé selon la revendication 4 ou 5, caractérisé en ce que les granules sont colorés par du bleu CIBACRON (Cibachronblau) 3 GA.